# EUROPEAN PATENT APPLICATION

(11) **EP 4 049 602 A1**
(43) Date of publication of application: **31.08.2022**
(21) Application number: 20878176.5
(22) Date of filing: 23.10.2020
(51) Int. Cl.: A61B 18/12

(54) **IN VIVO TEMPERATURE CONTROL SYSTEM**

(30) Priority: 24.10.2019 JP 2019193137
(71) Applicant: Toray Industries, Inc., Tokyo, 103-8666 (JP)
(72) Inventor: SAKAKIBARA, Hajime, Otsu-shi, Shiga 520-2141 (JP); OKADA, Tatsuya, Otsu-shi, Shiga 520-2141 (JP); NAKAJIMA, Hiroki, Otsu-shi, Shiga 520-2141 (JP); MATSUKUMA, Akinori, Otsu-shi, Shiga 520-2141 (JP); HARADA, Hiroyuki, Otsu-shi, Shiga 520-2141 (JP)
(74) Representative: Kador & Partner PartG mbB
(86) International application number: PCT/JP2020/039848
(87) International publication number: WO 2021/079974

(57) **Abstract**

The present invention provides an *in vivo* temperature control system having means for monitoring the internal temperature of a biological organ such as the esophagus, and for directly controlling the temperature in the organ side depending on the monitoring. The present invention provides an *in vivo* temperature control system comprising: a catheter insertable into a living body; a temperature probe containing a temperature sensor, the probe being insertable into the catheter; a liquid storage section for storing a temperature-controlled liquid; a pump for supplying the liquid from the liquid storage section to the catheter; and a control section for controlling driving of the pump based on a signal detected from the temperature probe; wherein the control section controls the pump when the signal has reached a preset threshold, and the pump is driven such that the liquid in the liquid storage section is released to the outside through the catheter.

## Description

### TECHNICAL FIELD

The present invention relates to an *in vivo* temperature control system.

### BACKGROUND ART

Atrial fibrillation is a kind of arrhythmia, and known to involve repeated irregular contraction of the atrium, leading to poor blood circulation, hence causing discomfort or malaise. Therefore, methods of treating atrial fibrillation have been widely carried out by catheter ablation procedures, wherein the pulmonary vein and the cardiac muscle tissues in the vicinity thereof such as the posterior wall of the left atrium, which are major sources of occurrence of atrial fibrillation, are ablated (pulmonary vein isolation).

On the other hand, since the site of ablation (left atrium) and the esophagus are positioned close to each other during the treatment by a catheter ablation procedure, it has been pointed out that there is a risk of injury of the esophagus, leading to severe esophageal complications such as left atrial-esophageal fistula and esophagus vagus nerve paralysis. Thus, appropriate control of the temperature in the esophagus is required.

Examples of means for the control of the temperature in the esophagus that have been reported include a temperature measurement device that employs an approach through the nose or mouth of the patient (nasal or oral approach) to insert a catheter equipped with a temperature sensor into the esophagus. The temperature sensor measures the internal esophageal temperature, and an alert is output to the outside when the internal temperature is judged to have reached a threshold (Patent Document 1).

Another device that has been reported comprises a temperature sensor and a controller that are directly connected to an ablation catheter for intracardiac ablation of a patient, wherein, when the internal esophageal temperature has increased to exceed an unacceptable level (threshold) or a predetermined value (temperature), the controller automatically controls the power output of the ablation catheter in order to prevent overheating and injury of the esophagus (Patent Document 2).

A heat-exchange balloon assembly that has been reported as a balloon catheter for cooling of the esophagus comprises an inflatable balloon which is suitable for insertion into the esophagus, which has a heat transfer surface on the outside, and whose inner lumen is suitable for retaining a heat exchange medium (Patent Document 3).

### PRIOR ART DOCUMENTS

### [Patent Documents]

[Patent Document 1] JP 2016-119936 A
[Patent Document 2] JP 2019-10500 A
[Patent Document 3] JP 2009-504284 A

### SUMMARY OF THE INVENTION

### PROBLEMS TO BE SOLVED BY THE INVENTION

The device described in Patent Document 1 is capable of monitoring the internal esophageal temperature during the ablation therapy and outputting the alert to the outside when the internal temperature is judged to have reached a threshold, to thereby allowing one to take measures such as stopping of the ablation before the esophagus is injured by heating or cooling. However, a delay in noticing of the alert may lead to a delay in the required process.

In the device described in Patent Document 2, the power output during the ablation is automatically controlled by the controller depending on the internal esophageal temperature. Therefore, the operator can concentrate on the manipulation of ablation. However, in the case where the power output during the ablation is controlled, the cooling of the esophagus is achieved by natural cooling, so that the cooling requires a long time, leading to placement of a significant burden on the patient. Moreover, since the device prevents overheating by the power output control for the ablation, it is not directly applicable to catheters that perform ablation by cooling, such as a cryoballoon.

The device described in Patent Document 3 is capable of preventing esophageal injury during the ablation procedure, by cooling the esophagus through a balloon placed in the esophagus. However, in order to prevent esophageal injury caused by thermal damage during the ablation procedure, the balloon needs to be inflated in a position of the esophagus close to the heart that is being ablated. This may make the esophagus even close to the ablation source, increasing the risk of the esophageal injury.

In view of this, an object of the present invention is to provide an *in vivo* temperature control system having means for monitoring the internal temperature of a biological organ such as the esophagus, and for directly controlling the temperature in the organ side depending on the monitoring.

### MEANS FOR SOLVING THE PROBLEMS

In order to solve the above problem, the present inventors intensively studied to discover the following inventions (1) to (7).
(1) An *in vivo* temperature control system comprising: a catheter insertable into a living body; a temperature probe containing a temperature sensor, the probe being insertable into the catheter; a liquid storage section for storing a temperature-controlled liquid; a pump for supplying the liquid from the liquid storage section to the catheter; and a control section for controlling driving of the pump based on a signal detected from the temperature probe; wherein the control section controls the pump when the signal reaches a preset threshold, and the pump is driven such that the liquid in the liquid storage section is released to the outside through the catheter.
(2) The *in vivo* temperature control system according to (1), wherein the control section controls the pump when a preset time or temperature is reached, and the pump is driven such that a liquid in the outside is sucked through the catheter.
(3) The *in vivo* temperature control system according to (1) or (2), comprising a pressure sensor for detecting the internal pressure of the catheter, wherein the control section controls driving of the pump based on a signal detected by the pressure sensor.
(4) The *in vivo* temperature control system according to any one of (1) to (3), wherein the control section drives the pump such that the rotation speed during the suction of the liquid is lower than the rotation speed during the sending of the liquid.
(5) The *in vivo* temperature control system according to any one of (1) to (4), comprising a monitor for displaying the signal detected from the temperature probe, as a digital number, bar graph, or trend graph, the system having means for allowing an operator to know that the signal detected from the temperature probe exceeded the preset threshold, by way of changing display color of the digital number, bar graph, or trend graph on the monitor.
(6) The *in vivo* temperature control system according to any one of (1) to (5), wherein the pump is a liquid-sending pump and a suction pump, and the control section drives the liquid-sending pump when the liquid is to be sent, and drives the suction pump when the liquid is to be sucked.
(7) A method of controlling an *in vivo* temperature control system, the *in vivo* temperature control system comprising: a catheter insertable into a living body; a temperature probe containing a temperature sensor, the probe being insertable into the catheter; a liquid storage section for storing a temperature-controlled liquid; a pump for supplying the liquid from the liquid storage section to the catheter; and a control section for controlling driving of the pump based on a signal detected from the temperature probe; the method comprising the steps of: making the control section compare the signal detected from the temperature probe with a preset threshold; and making, when the signal is judged to have reached the threshold, the control section drive the pump such that the liquid in the liquid storage section is released to the outside through the catheter.

### EFFECT OF THE INVENTION

According to the present invention, a temperature probe measures the internal temperature of a biological organ, and, when a control section senses that a preset temperature is reached, the control section drives a pump to automatically release a liquid from a liquid storage section to the outside of a catheter, to enable control of the internal temperature of the biological organ to a predetermined temperature using the liquid. Therefore, for example, in treatment of arrhythmia by an ablation procedure, the internal esophageal temperature during the ablation procedure can be appropriately controlled by automatically sending the liquid into the esophagus in accordance with the internal esophageal temperature.

### BRIEF DESCRIPTION OF THE DRAWINGS

Fig. 1 is diagram illustrating the external appearance of an *in vivo* temperature control system according to a first embodiment of the present invention.
Fig. 2 is a schematic diagram illustrating the internal structure of the *in vivo* temperature control system illustrated in Fig. 1.
Fig. 3 is schematic diagram illustrating the internal structure of an *in vivo* temperature control system according to a second embodiment of the present invention.
Fig. 4 is a schematic diagram illustrating a more concrete configuration of a liquid-sending-sucking dual-purpose tube in the *in vivo* temperature control system of Fig. 2.
Fig. 5 is a schematic diagram illustrating the flow of the liquid during liquid sending by the *in vivo* temperature control system of Fig. 2.
Fig. 6 is a schematic diagram illustrating the flow of the liquid during suction by the *in vivo* temperature control system of Fig. 2.
Fig. 7 is a time chart illustrating a first control procedure of an *in vivo* temperature control system of the present invention.
Fig. 8 is a flow chart illustrating an operational procedure of the control section in the first control operation of the *in vivo* temperature control system of the present invention.
Fig. 9 is a time chart illustrating a second control operation in an *in vivo* temperature control system.
Fig. 10 is a flow chart illustrating an operational procedure of the control section in the second control operation of the *in vivo* temperature control system of the present invention.
Fig. 11 is a diagram illustrating the results of temperature measurement using an *in vivo* temperature control system of the present invention.

### MODE FOR CARRYING OUT THE INVENTION

Specific embodiments of the present invention are described below with reference to drawings. However, the present invention is not limited to these modes. The present invention may be modified as appropriate without departing from the scope in which the effect of the invention can be produced. It should be noted that the same reference numerals are used for the same components.

### <First Embodiment>

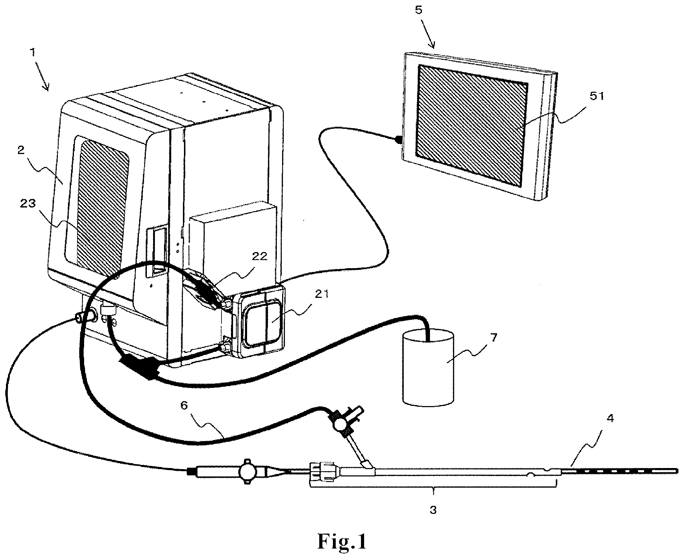

Fig. 1 is a diagram illustrating the external appearance of an *in vivo* temperature control system 1 according to a first embodiment of the present invention. For example, when an ablation procedure is carried out using a balloon ablation catheter in which the inside of the balloon is heated using a raciofrequency, the *in vivo* temperature control system 1 may be used for monitoring the internal esophageal temperature in a position close to the heart to be treated by the ablation, and also for cooling the internal esophageal temperature with a liquid. The biological organ to which the *in vivo* temperature control system 1 is applicable is not limited. The system may be applied to the pharynx, larynx, lung, esophagus, stomach, or the like. It is especially preferably used for cooling of the inside of the esophagus.

The *in vivo* temperature control system 1 herein comprises: an *in vivo* temperature control device 2 for sending or sucking a temperature-controlled liquid to a catheter 3; a catheter 3 on which a pore(s) through which a liquid can be sent into or sucked from a living body is/are formed, the catheter being insertable into the living body; a temperature probe 4 containing a temperature sensor, the probe being insertable into the catheter 3; a monitor 5 capable of displaying a signal from the temperature probe 4; a liquid-sending-sucking dual-purpose tube 6 connected to a pump 21 and a pressure center 22, the tube connecting the *in vivo* temperature control device 2 to the catheter 3; and a waste liquid section 7.

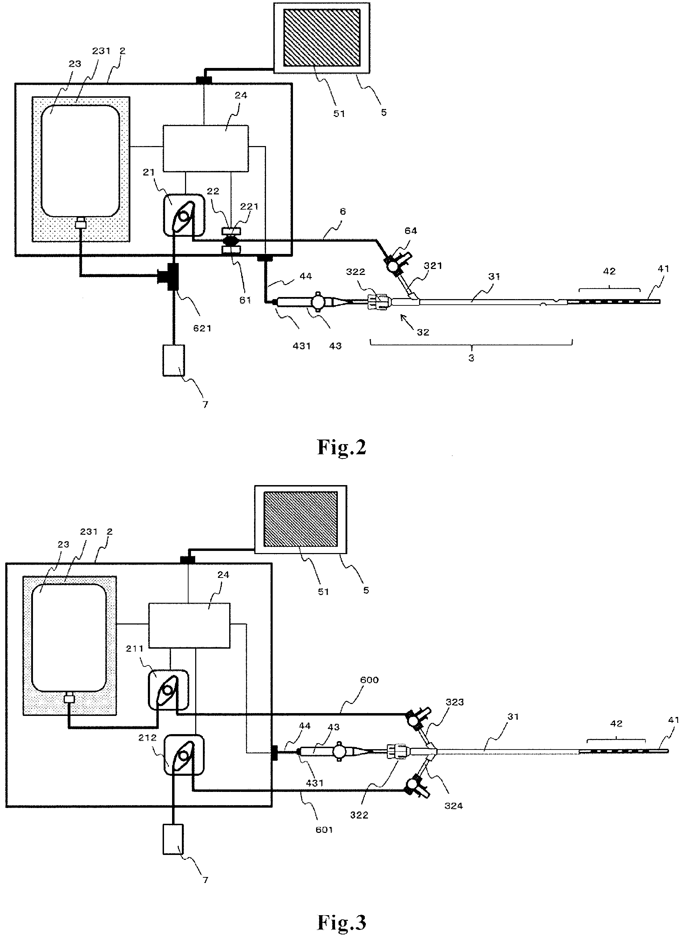

Fig. 2 is a schematic diagram illustrating the internal structure of the *in vivo* temperature control device 2 illustrated in Fig. 1.

The *in vivo* temperature control device 2 comprises: a pump 21 for sending or sucking a liquid to the catheter 3; a pressure sensor 22 for detecting the internal pressure of the catheter 3; a liquid storage section 23 for storing the temperature-controlled liquid; and a control section 24 for controlling driving of the pump based on a signal detected from the temperature probe 4 or a signal detected from the pressure sensor 22.

The pump 21 contained in the *in vivo* temperature control device 2 is a roller-type tube pump. By the forward rotation of the roller of the pump 21, a liquid can be sent from the liquid storage section 23 to the catheter 3. By the reverse rotation of the roller of the pump 21, a liquid can be sucked from the distal end portion or the pore(s) of the catheter 3.

The pump driving method used for the pump 21 is not limited. For maintaining the cleanness of the liquid to be sent to or sucked from the living body, and for simply controlling the flow rate of the liquid, a tube pump is preferably used. A roller-type or finger-type tube pump is more preferred.

The *in vivo* temperature control device 2 may comprise a plurality of pumps. For example, as an embodiment other than the first embodiment, an *in vivo* temperature control device 2 comprising two pumps is illustrated in Fig. 3. In this case, the *in vivo* temperature control device 2 separately comprises a liquid-sending pump 211 and a suction pump 212. In cases where the catheter 3 is a multilumen catheter comprising: a liquid-sending lumen; and a suction lumen for sucking a liquid; and where the liquid-sending pump 211 and the suction pump 212 arc connected to ports leading to the respective lumens, the liquid-sending operation and the suction operation can be simultaneously carried out. Since the above configuration enables continuous sending of the liquid into the living body, and also enables discharge of the heat-exchanged liquid to the outside of the body, the temperature control in the living body can be efficiently carried out.

The pressure sensor 22 included in the *in vivo* temperature control device 2 comprises a contact-type displacement gauge 221, and a bulge section 61 of the liquid-sending-sucking dual-purpose tube 6 in contact with the contact-type displacement gauge 221. The bulge section 61 of the liquid-sending-sucking dual-purpose tube 6 is a portion prepared by forming part of the liquid-sending-sucking dual-purpose tube 6 into a bag shape. The bulge section 61 is designed such that it expands or shrinks in accordance with the pressure in the tube. Thus, by detecting the amount of displacement of the bulge section 61 as a signal by the contact-type displacement gauge 221, the internal pressure of the catheter 3 connected to the liquid-sending-sucking dual-purpose tube 6 can be measured through the liquid-sending-sucking dual-purpose tube 6.

The detection method by the pressure sensor 22 is not limited. Any detection method may be employed as long as the internal pressure of the catheter 3 during the liquid sending or sucking operation can be detected and sent to the control section 24 as a signal. For example, as an embodiment other than the first embodiment, a method in which a diaphragm-type inline pressure sensor is added, or a method in which a thin tube is branched from the liquid-sending-sucking dual-purpose tube 6, and the pressure in the thin tube is measured, may be employed.

The liquid storage section 23 of the *in vivo* temperature control system 1 of the first embodiment is a general, commercially available infusion bag for physiological saline, glucose solution, or the like. The liquid storage section 23 has a structure by which it can be housed inside the *in vivo* temperature control device 2. In the *in vivo* temperature control device 2, a Peltier device 231 and an in-device temperature sensor, which is not shown, for measurement of the temperature in the liquid storage section 23 are placed such that they are in contact with the liquid storage section 23. By controlling the temperature of the Peltier device 231 based on a signal detected from the in-device temperature sensor, temperature control of the liquid stored in the liquid storage section 23 can be carried out. In cases where a catheter ablation procedure using a hot balloon is carried out, the temperature of the liquid in the liquid storage section 23 is controlled preferably at 0°C to 15°C, more preferably at 0°C to 10°C.

The liquid storage section 23 may be in any shape as long as it is capable of storing a liquid such as physiological saline. The liquid storage section 23 may be housed inside the *in vivo* temperature control device 2, or may be placed outside the *in vivo* temperature control device 2. In cases where the liquid storage section 23 is housed inside the *in vivo* temperature control device 2, temperature control of the liquid in the liquid storage section 23 is preferably possible as described above. In an embodiment other than the first embodiment, the infusion bag may be cooled using a coolant such as ice instead of the Peltier device 231, or an infusion bag that has been preliminarily frozen may be used while thawing it. The liquid used may be purified water or tap water instead of the physiological saline or glucose solution.

In cases of use in cryoablation, and in cases of use in high-frequency hyperthermia for cancer therapy (especially for pharyngeal cancer, laryngeal cancer, lung cancer, esophagus cancer, or stomach cancer), the inside of the living body needs to be warmed to a higher level than usual. The *in vivo* temperature control system of the present invention may be used therefor. In such cases, the liquid stored in the liquid storage section 23 may be heated to a temperature of 30 to 45°C using a heating resistor.

The control section 24 contained in the *in vivo* temperature control device 2 controls the pump 21. It drives the pump 21 based on a signal detected from the temperature probe 4, such that the liquid in the liquid storage section 23 is released to the outside through the catheter 3. More specific control operation is described later. Further, the control section 24 contained in the *in vivo* temperature control device 2 preferably comprises a circuit for controlling driving of the pump 21 based on a signal detected by the pressure sensor 22. For example, the control section 24 of the *in vivo* temperature control system 1 according to the first embodiment has a mechanism that allows numerical conversion of the information from the liquid-sending-sucking dual-purpose tube 6 on the amount of displacement to pressure information.

Further, the control section 24 preferably comprises a circuit for controlling the internal temperature of the liquid storage section 23 based on a signal detected from the in-device temperature sensor for measurement of the temperature inside the liquid storage section 23.

The catheter 3 is a cylindrical member insertable into a living body by a nasal or oral approach, and capable of sending or sucking a liquid through the distal end of the catheter or a pore(s) formed on the surface, through a lumen. More specifically, the catheter 3 has a structure comprising a tube section 31 insertable into a living body, and a valved connector 32 fixed at the proximal end side in the longitudinal direction of the tube section 31.

The material used for the tube section 31 is not limited as long as it is a flexible material nasally or orally insertable into a living body, and examples of the material include thermoplastic resins such as polyvinyl chloride, polyurethane, and silicone. For the confirmation of the site of placement in the living body, the material preferably contains a radiopaque material.

For example, in cases where the tube section 31 is nasally inserted into the living body from the nose, the length of the tube section 31 is preferably about 200 mm to 1000 mm; the outer diameter is preferably about 1.7 mm to 6.0 mm; and the inner diameter is preferably about 1.0 mm to 5.0 mm.

The valved connector 32 is fixed at the proximal end side of the tube section 31, and connectable to the liquid-sending-sucking dual-purpose tube 6. The valved connector 32 comprises a port 321 for sending or sucking of the liquid from the distal end side of the tube section 31, and a valve 322 for fixing the temperature probe 4 in the case where the temperature probe 4 is inserted to the catheter 3. The valve 322 is preferably openable and closable by a rotational motion or the like. The above configuration enables manipulation of the temperature probe 4 when the valve 322 is open, and enables fixation of the temperature probe 4 when the valve 322 is closed.

In an embodiment other than the first embodiment, the valved connector 32 of the catheter 3 may comprise a first port 323 and a second port 324 as illustrated in Fig. 3. By connecting a liquid-sending tube 600 connected to a liquid storage section 23 and a liquid-sending pump 212 to the first port 323, and connecting a suction tube 601 connected to a suction pump 212 and a waste liquid section 7 to the second port 324, a liquid-sending line and a suction line can be independently provided. By this, a cooled liquid can be constantly sent into the living body.

The catheter 3 may be a multilumen catheter comprising a liquid-sending lumen for sending a liquid and a suction lumen for sucking a liquid, wherein the first port 323 may be a liquid-sending port leading to the liquid-sending lumen, and wherein the second port 324 may be a suction port leading to the suction lumen. By this, the liquid-sending operation and the suction operation can be simultaneously carried out.

The temperature probe 4 is a member to be nasally or orally inserted into a living body to measure the internal temperature of a biological organ. The temperature probe 4 comprises a shaft section 41 to be inserted into the living body, a temperature sensor 42 placed at the distal end side, and a handle section 43.

The material used for the shaft section 41 is not limited as long as it is a flexible material nasally or orally insertable into a living body, and examples of the material include thermoplastic resins such as polyether block amide, polyurethane, nylon, polyolefin, polyamide, and polyetheramide.

The outer diameter of the shaft section 41 is preferably about 1.0 mm to 4.0 mm, more preferably a diameter that allows insertion of the shaft section 41 into a lumen of the catheter 3. The shaft section 41 preferably has a length of about 300 mm to 1100 mm. In cases where it is inserted into a lumen of the catheter 3, the temperature sensor 42 on the shaft section 41 is preferably placed at a position where it protrudes from the distal end side of the catheter 3.

The shaft section 41 may have a function by which the distal end side can be deflected by operation of the handle section 43. By this, in particular, in cases of application to the esophagus, the risk of straying into the airway can be reduced when the temperature probe 4 is nasally or orally inserted into the esophagus. Moreover, the esophagus is meandering, rather than being straight, between the pharynx and the gastric cardia. By the deflection operation, placement of the temperature sensor 42 at a desired esophageal site is possible.

One or more temperature sensors 42 are attached to the distal end side of the shaft section 41. In order to allow measurement of the internal temperature in a larger area in the biological organ, a plurality of temperature sensors 42 are preferably contained.

The material used for the temperature sensor 42 is not limited as long as it has good thermal conductivity. It is preferably a radiopaque material from the viewpoint of measurement of the temperature at a position close to the ablation site.

The handle section 43 comprises a connector 431 for connection to the *in vivo* temperature control device 2. In the *in vivo* temperature control system 1 according to the first embodiment, the *in vivo* temperature control device 2 is connected to the temperature probe 4 through a connection cable 44.

The monitor 5 is capable of displaying information on the internal temperature in the living body detected by the temperature probe 4, as a digital number, bar graph, or trend graph. The monitor 5 has a function by which, when the temperature in the biological organ has exceeded a preset threshold, the display color is changed to present the temperature change to an operator as visual information. For example, the monitor may be set such that the color changes from a cold color to a warm color as the temperature increases from low temperature to high temperature. By this, the temperatures detected by the temperature probes 4 placed along the longitudinal direction in the living body can be known as the temperatures at the corresponding positions in the living body. Therefore, the operator can perceive which site of the biological organ has higher temperature than the others. Further, since the temperature is presented not only as a digital number, but also as a bar graph, the temperature can be compared among different sites in the living body. Further, the operator can be visually informed of the fact that the internal temperature of the living body has increased to a level at which the risk of injury of the biological organ is high.

The monitor 5 preferably has a function for presenting, to the operator, information on the operation of sending and sucking of the liquid; information on errors that have occurred in the system, and alerts including alarms; and operational information such as the operation time, the numbers of times of sending and sucking of the liquid, and the amount of liquid sent. By this, the operational conditions, malfunctioning conditions, and dangerous conditions can be visually or aurally informed to the operator.

The monitor 5 preferably comprises a touch-screen display 51 with which various parameters related to the system operation can be input, and with which the input parameters can be transmitted to the control section 24 of the *in vivo* temperature control device 2. This enables the operator to start and stop the operation, and to set and modify various parameters, of the *in vivo* temperature control device 2 from a distant location.

The liquid-sending-sucking dual-purpose tube 6 is a tube for delivering a liquid from the liquid storage section 23 to the catheter 3 through the pump 21 during the sending of the liquid, and delivering a liquid from the catheter 3 to the waste liquid section 7 during the suction of the liquid. The waste liquid section 7 is a site for storing the unnecessary liquid after the suction of the liquid from the body.

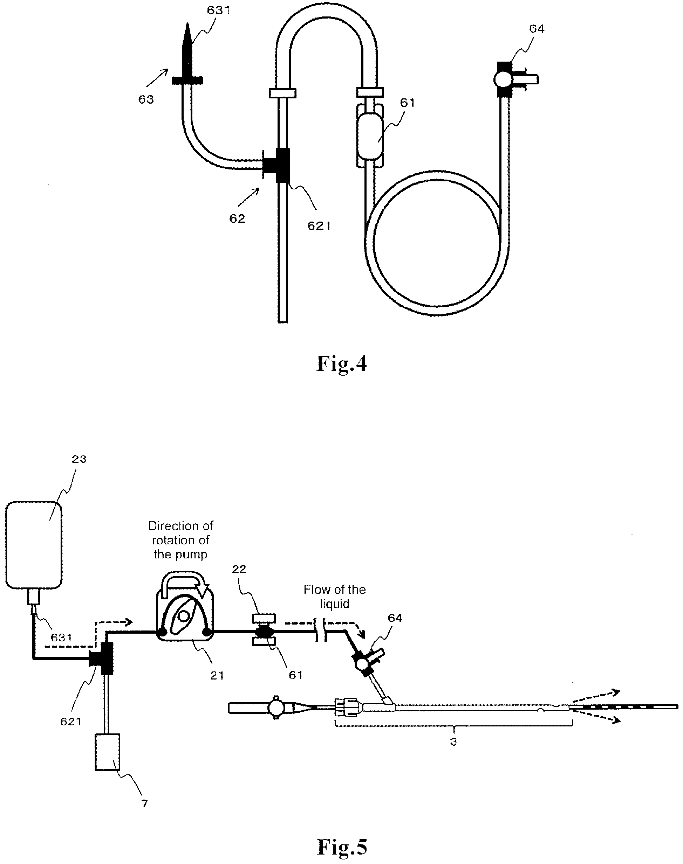

As illustrated in Fig. 4, the liquid-sending-sucking dual-purpose tube 6 contained in the *in vivo* temperature control system 1 comprises a bulge section 61, a channel switching section 62, a liquid supply port 63 for connection to the liquid storage section 23, and a connection port 64 for connection to the catheter 3.

As described above, the bulge section 61 is a tube formed into a bag shape, and designed such that it expands or shrinks in accordance with the pressure in the tube. Thus, by detecting the amount of displacement of the bulge section 61 by the contact-type displacement gauge 221, the internal pressure of the catheter 3 connected to the liquid-sending-sucking dual-purpose tube 6 can be measured through the liquid-sending-sucking dual-purpose tube 6.

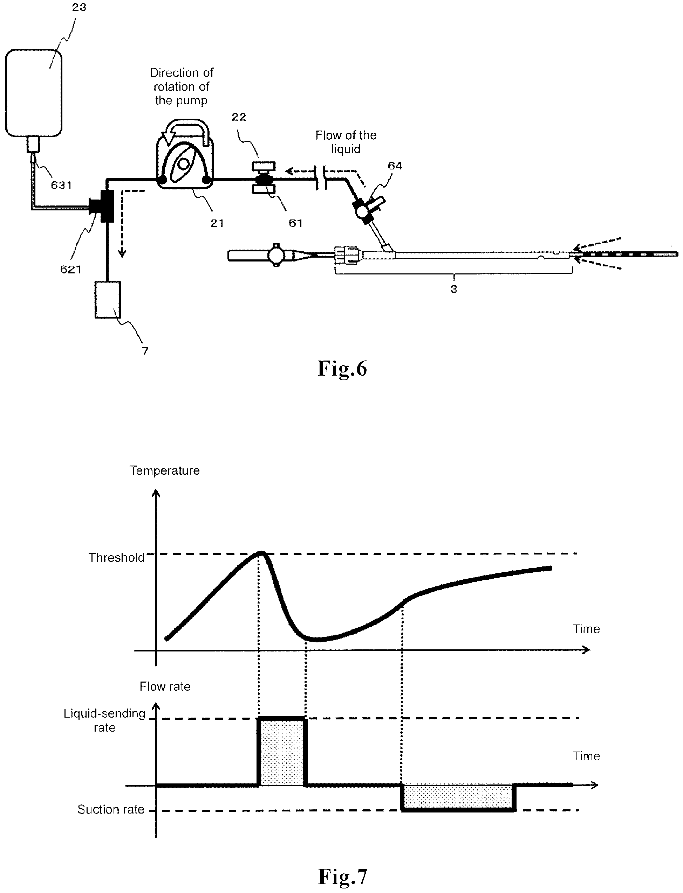

The channel switching section 62 in the first embodiment is a three-way check valve 621, and connected to the primary side of the pump 21. Therefore, as described in Fig. 5, when the liquid is to be sent, forward rotation of the pump 21 switches the channel of the three-way check valve 621 to the direction in which the liquid storage section 23 is connected to the pump 21, allowing the liquid to flow to the catheter 3. Further, as described in Fig. 6, when the liquid is to be sucked from the catheter 3, reverse rotation of the pump 21 switches the channel of the three-way check valve 621 to the direction in which the pump 21 is connected to the waste liquid section 7, allowing the sucked liquid to be discharged to the waste liquid section 7. Each of the dotted-line arrows and the solid-line arrows in Fig. 5 and Fig. 6 represents the direction of rotation of the pump 21 or the flow of the liquid.

In an embodiment other than the first embodiment, as the channel switching section 62, a liquid-sending-sucking dual-purpose tube with T-shaped branching and two pinch valves may be used instead of the three-way check valve. Preferably, in this case, the *in vivo* temperature control device 2 additionally comprises the pinch valves, and the control section 24 controls switching of the channels by opening and closing the pinch valves in the *in vivo* temperature control device 2 in accordance with the pump driving.

The liquid supply port 63 may be in any form as long as the liquid can be supplied from the liquid storage section 23 into the liquid-sending-sucking dual-purpose tube 6. In the first embodiment, since the liquid storage section 23 is an infusion bag, the liquid supply port 63 is preferably a needle 63 1 capable of piercing the infusion bag.

The connection port 64 may be in any form as long as it can be connected to the catheter 3. It is preferably a three-way stopcock. In this case, when malfunction of the *in vivo* temperature control system occurs, a syringe or the like can be connected so as to enable manual sending and sucking of the liquid.

The following describes, using Fig. 7, a time chart illustrating a first control operation of the *in vivo* temperature control system for maintaining the internal esophageal temperature constant during treatment of arrhythmia by a catheter ablation procedure.

### Step 1: Process of Comparison of Temperature Information with Threshold

After a catheter ablation procedure for ablation of the cardiac tissue begins in a position close to the left atrium, the internal esophageal temperature gradually increases in the vicinity thereof, and the internal esophageal temperature as measured by each temperature sensor 42 of the temperature probe 4 also gradually increases. In the control section 24, a threshold of the internal esophageal temperature at which the sending of the cooled liquid (cooling water) is begun can be preliminarily set, and the process of comparing the temperature information of the temperature sensor 42 with the threshold is constantly carried out.

### Step 2: Operation of Sending Liquid (Cooling Water)

When the temperature information from at least one of the pluralities of temperature sensors 42 has reached the threshold, the control section 24 outputs a driving command regarding the liquid-sending rate and the liquid-sending time to the pump 21. As a result, the liquid (cooling water) is sent from the liquid storage section 23 to the catheter 3 through the liquid-sending-sucking dual-purpose tube 6. When the liquid (cooling water) reaches the site at an increased temperature in the esophagus, the inside of the esophagus can be cooled by heat exchange with the liquid (cooling water). The amount of the liquid (cooling water) sent is controlled based on the liquid-sending rate and the liquid-sending time, which may be preset in the control section 24. The liquid (cooling water) is preferably sent in a short time. More specifically, the liquid is preferably sent at a liquid-sending rate of 1 mL/min to 300 mL/min.

### Step 3: Operation of Keeping Liquid (Cooling Water) in Esophagus

The operation time of Step 3 may be preset in the control section 24 such that Step 4 is begun after the set time has passed. In another method that may be employed, the internal esophageal temperature is monitored, and Step 4 is begun at the time when the internal esophageal temperature has reached a preset temperature.

### Step 4: Operation of Suction of Liquid Sent into Esophagus

For preventing aspiration caused by flowing out of the sent liquid into the airway, the system is preferably capable of sucking the whole amount of the sent liquid. Further, since suction of the liquid in a short time may cause esophageal injury due to excessive suction, the suction rate is preferably set to a rate lower than the liquid-sending rate. More specifically, the suction is preferably carried out at a suction rate of 1 mL/min to 100 mL/min. The suction time may be preset in the control section 24. The suction time in which the whole amount of the sent liquid can be sucked may be calculated by dividing the amount of the sent liquid by the suction rate. Further, the suction rate may be calculated by dividing the amount of the liquid sent or sucked by the suction time. Thus, various modifications are possible therefor.

In another suction method, a pressure change in the liquid-sending-sucking dual-purpose tube 6 may be used. More specifically, in cases where the suction is continued after sucking the whole amount of the liquid from the esophagus, the esophagus becomes flat. This results in occlusion of the catheter 3, so that the internal pressure of the liquid-sending-sucking dual-purpose tube 6 becomes negative. Thus, by detecting the internal pressure of the liquid-sending-sucking dual-purpose tube 6 by the pressure sensor 22, and sending the signal to the control section 24, the control section 24 can stop driving of the pump 21 at the time when the whole amount of the liquid has been sucked or when the liquid in the esophagus has become absent. More specifically, the suction operation is preferably stopped when the internal pressure of the liquid-sending-sucking dual-purpose tube 6 is within the range of -20 kPaG to -90 kPaG.

After the operation of Step 4, the process proceeds to Step 1 again, and then Steps 1 to 4 are repeated. By this, the esophageal temperature can be appropriately maintained. Since the first control operation is a control operation for cases where the internal esophageal temperature increases due to ablation of cardiac muscle by a radiofrequency ablation procedure or the like, the circuit is prepared such that the control section 24 judges reaching of the threshold when the esophageal temperature has exceeded the threshold. However, in cases where the internal esophageal temperature decreases due to a cryoablation procedure or the like, the internal esophageal temperature can be controlled by an operation which is the same as the second control operation described above except that heated water is used as the liquid to be sent, and that the circuit is prepared such that the control section 24 judges reaching of the threshold when the esophageal temperature has become lower than the threshold.

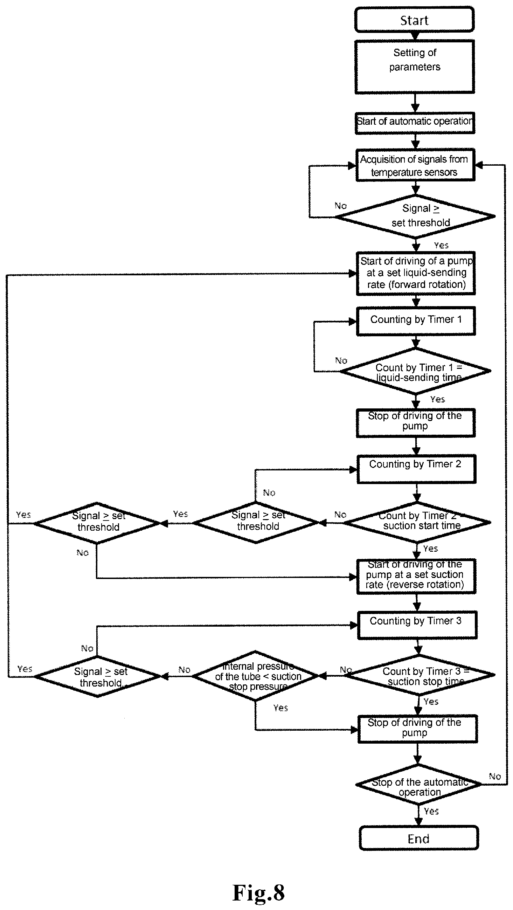

A flow chart illustrating an example of the operational procedure for the control section 24 in the first control method is described below using Fig. 8.

In this example of the operational procedure, first, an operator operates the touch-screen display 51 of the monitor 5 connected to the *in vivo* temperature control device 2 to preset, in the control section 24, any necessary value(s) selected from the liquid-sending start temperature, the amount of liquid sent, the liquid-sending rate, the suction start temperature, the suction start time, the amount of suction, the suction rate, the suction stop time, and the suction stop pressure.

Subsequently, when the operator starts automatic operation, the control section 24 detects a signal(s) (such as the thermoelectromotive force) from the temperature sensor(s) 42 in the temperature probe 4 connected to the *in vivo* temperature control device 2, and then converts the signal(s) to temperature information (*in vivo* temperature(s)).

Subsequently, the control section 24 judges whether or not at least one temperature in the temperature information acquired from the temperature sensor(s) 42 has reached the preset threshold of the liquid-sending start temperature, in other words, whether or not the condition for the start of sending of the liquid (cooling water) is satisfied, by comparison of the values. In cases where the control section 24 has judged that at least one *in vivo* temperature has reached the preset threshold of the liquid-sending start temperature, the control section 24 drives the pump 21 (forward rotation) to start sending of the liquid (cooling water) from the liquid storage section 23. After the start of sending of the liquid (cooling water), counting by a timer (Timer 1) is started upon the driving of the pump 21, and the liquid (cooling water) is continuously sent until the liquid-sending time, calculated from the preset amount of the liquid to be sent and the preset liquid-sending rate, is reached. After the calculated liquid-sending time is reached, the control section 24 stops the driving of the pump 21.

On the other hand, when none of the *in vivo* temperatures is judged to have reached the liquid-sending start temperature, the temperature is within a normal temperature range that does not require liquid sending for the temperature control. Thus, the control section 24 continues to acquire the *in vivo* temperatures to judge whether or not the condition for the start of sending of the liquid (cooling water) is satisfied.

After the completion of sending of the liquid (cooling water), the control section 24 starts counting by a timer (Timer 2), and judges whether or not the count by Timer 2 reached the preset threshold of the suction start time, in other words, whether or not a first condition for the start of suction is satisfied. Here, in cases where the first condition for the start of suction is judged to have been satisfied, the control section 24 drives the pump 21 (reverse rotation) to start suction of the liquid from the living body. On the other hand, in cases where the count by Timer 2 is judged not to have reached the suction start time, the control section 24 subsequently judges whether or not at least one *in vivo* temperature acquired has reached the preset threshold of the suction start temperature or higher, in other words, whether or not a second condition for the start of suction is satisfied. In the second condition for the start of suction, in cases where the *in vivo* temperature is less than the preset threshold of the suction start temperature, the count by Timer 2 is started again to judge whether or not the first condition for the start of suction is satisfied. On the other hand, in cases where the *in vivo* temperature is judged to have reached the preset threshold of the suction start temperature, the control section 24 judges whether or not the liquid-sending start condition is satisfied.

In the liquid-sending start condition, when at least one *in vivo* temperature has reached the preset threshold of the liquid-sending start temperature or higher, the control section 24 drives the pump 21 (forward rotation) to start sending of the liquid from the liquid storage section 23 in order to control the *in vivo* temperature within a normal temperature range. On the other hand, in cases where the *in vivo* temperature is less than the preset threshold of the liquid-sending start temperature, the control section 24 drives the pump 21 (reverse rotation) to start suction of the liquid from the inside of the living body.

After the completion of the sending of the liquid, the control section 24 starts counting by Timer 2, and repeats the liquid-sending operation until the first condition for the start of the suction and the second condition for the start of the suction are satisfied. In cases where the liquid sending has been repeatedly carried out without starting the suction, there is an increased risk of pulmonary aspiration caused by excessive administration of the liquid. Therefore, in this case, it is preferred to sound an alarm to inform the operator of the abnormality. In cases where the liquid-sending start condition is judged not to have been satisfied, the control section 24 continues to judge whether or not the suction start condition is satisfied, and then to judge whether or not the liquid-sending start condition is satisfied.

After the start of the suction, counting by a timer (Timer 3) is started upon the driving of the pump 21. This is followed by judgement on whether or not the count by Timer 3 has reached the preset suction stop time, in other words, whether or not a first condition for the stop of suction is satisfied. The suction stop time may be determined by means of calculation using the amount of suction and the suction rate that are preset. In cases where Timer 3 is judged to have reached the suction time, the control section 24 stops the driving of the pump 21. On the other hand, in cases where the suction time is judged not to have been reached, the control section 24 subsequently calculates the internal tube pressure based on a signal detected by the pressure sensor 22, and judges whether or not the internal tube pressure is not more than the preset threshold of the suction stop pressure, in other words, whether or not a second condition for the stop of suction is satisfied. In cases where the internal tube pressure is judged to be not more than the preset threshold of the suction stop pressure, the control section 24 stops the driving of the pump 21.

In cases where the internal tube pressure is judged not to have reached the preset threshold of the suction stop internal tube pressure or less, the control section 24 judges whether or not the condition for the start of sending of the liquid (cooling water) is satisfied. Here, in cases where at least one *in vivo* temperature is judged to satisfy the liquid-sending start condition, the control section 24 changes the driving of the pump 21 from reverse rotation to forward rotation to start sending of the liquid (cooling water) from the liquid storage section 23 in order to control the *in vivo* temperature within a normal temperature range. On the other hand, in cases where the liquid-sending start temperature is judged not to have been reached, the control section 24 repeatedly judges whether or not the suction stop condition is satisfied, and then whether or not the liquid-sending start condition is satisfied.

After the completion of the suction of the liquid, the control section 24 judges again whether or not the condition for the start of sending of the liquid (cooling water) is satisfied, and controls the *in vivo* temperature according to the above-described flow until the operator stops the automatic operation.

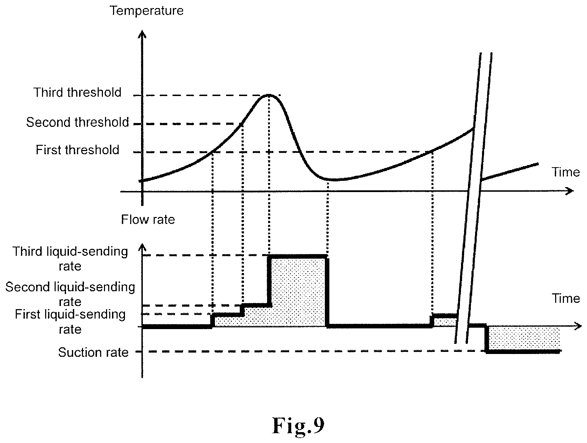

The following describes, using Fig. 9, a time chart illustrating a second control method as another control method for the *in vivo* temperature control system to maintain the esophageal temperature constant.

A plurality of thresholds of the esophageal temperature and their corresponding liquid-sending rates can be preset in the control section 24, so that the cooled liquid (cooling water) can be continuously sent in accordance with the set temperatures. Although a case designed to have three stages is illustrated in Fig. 9, the number of stages may be arbitrarily set by the operator.

### Step 1: Process of Comparison of Temperature Information with Threshold

After a catheter ablation procedure for ablation of the cardiac tissue begins in a position close to the left atrium, the esophageal temperature gradually increases in the vicinity thereof, and the esophageal temperature as measured by each temperature sensor 42 of the temperature probe 4 also gradually increases. The control section 24 constantly carries out the process of comparing the temperature information from each temperature sensor 42 with the plurality of thresholds.

### Step 2: Operation of Sending Liquid (Cooling Water)

When the temperature information measured by at least one of the temperature sensors 42 reaches a first esophageal temperature threshold (first threshold), the control section 24 outputs a driving command for a first liquid-sending rate to the pump 21. As a result, a cooled liquid (cooling water) is sent from the liquid storage section 23 to the catheter 3 through the liquid-sending-sucking dual-purpose tube 6. Further, when the catheter ablation procedure proceeds to increase the internal esophageal temperature, and the temperature information on the internal esophagus temperature(s) measured at at least one position reaches a second esophageal temperature threshold (second threshold) as a result, the control section 24 outputs a driving command for a second liquid-sending rate to the pump 21. Thereafter, depending on the increase in the esophageal temperature, the process proceeds to a third setting.

In cases where the internal esophageal temperature has become lower than the Nth temperature threshold by the sending of the liquid (cooling water), the system is controlled such that a driving command for the (N-1)th liquid-sending rate is output to the pump 21. In cases where the temperature has become lower than the first threshold, the sending of the liquid (cooling water) is stopped. By repeating the control of Step 2 during the ablation procedure, the liquid (cooling water) can be sent at appropriate flow rates in accordance with increases and decreases in the internal esophageal temperature, so that the inside of the esophagus can be more effectively cooled.

In this process, the rate of sending of the liquid (cooling water) preferably increases as the esophageal temperature increases as follows: first liquid-sending rate < second liquid-sending rate <... < Nth liquid-sending rate. More specifically, the liquid-sending rate is preferably set within the range of 1 to 300 mL/min. More preferably, the liquid-sending rate is set to a high rate (more specifically, 200 mL/min to 300 mL/min) when the internal esophageal temperature has exceeded a threshold (which is set to, for example, a dangerous internal temperature such as 40°C), and the liquid-sending rate before the exceeding of the threshold is set to a low rate (more specifically, 1 mL/min to 50 mL/min). By this, the risk of aspiration caused by excessive administration of the liquid (cooling water) can be reduced.

### Step 3: Operation of Suction of Liquid Sent into Esophagus

For continuously preventing aspiration caused by the sent liquid, it is preferred to carry out a sucking operation when the cumulative amount of the liquid sent has exceeded a set amount. The cumulative amount of the sent liquid at which the suction of the liquid is begun may be preset in the control section 24 as the suction-starting cumulative amount of the sent liquid. Further, as in the first control method, since suction of the liquid in a short time may cause esophageal injury due to excessive suction, the suction rate is preferably set to a lower rate than the liquid-sending rate.

In another suction method, the pressure change in the liquid-sending-sucking dual-purpose tube 6 may be used. More specifically, in cases where the suction is continued after sucking the whole amount of the liquid from the esophagus, the esophagus becomes flat. This results in occlusion of the catheter 3, and the internal pressure of the liquid-sending-sucking dual-purpose tube 6 becomes negative. Thus, by detecting the internal pressure in the liquid-sending-sucking dual-purpose tube 6 by the pressure sensor 22, and sending the signal to the control section 24, the control section 24 can stop the driving of the pump 21 at the time when the whole amount of the liquid has been sucked or when the liquid in the esophagus has become absent. More specifically, the suction operation is preferably stopped when the internal pressure of the liquid-sending-sucking dual-purpose tube 6 is within the range of -20 kPaG to -90 kPaG.

After the operation of Step 3, the process proceeds to Step 1 again, and then Steps 1 to 3 are repeated. By this, the esophageal temperature can be appropriately maintained. Since the second control method is a control method for cases where the internal esophageal temperature increases due to ablation of cardiac muscle by a radiofrequency ablation procedure or the like, the circuit is prepared such that the control section 24 judges reaching of the threshold when the esophageal temperature has exceeded the threshold. However, in cases where the internal esophageal temperature decreases due to a cryoablation procedure or the like, the internal esophageal temperature can be controlled by an operation which is the same as the second control method described above except that heated water is used as the liquid to be sent, and that the circuit is prepared such that the control section 24 judges reaching of the threshold when the esophageal temperature has become lower than the threshold.

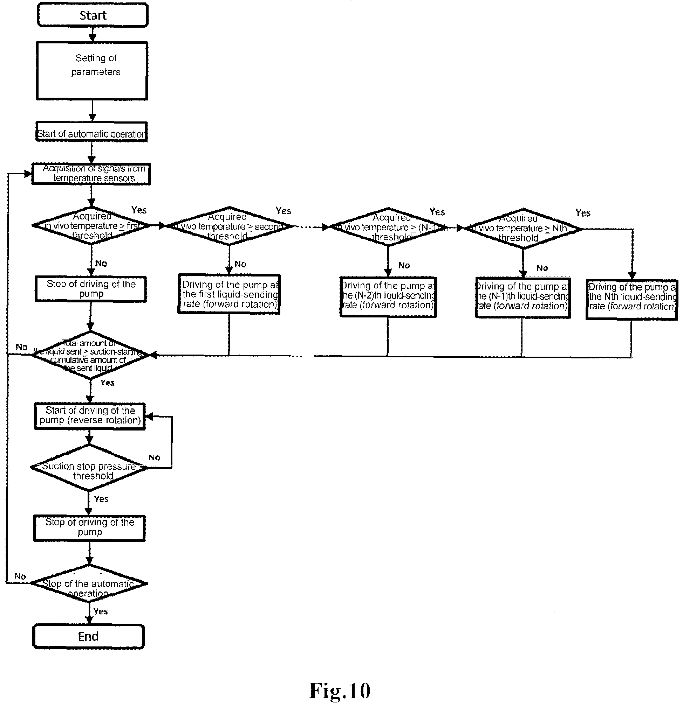

A flow chart illustrating an operational procedure for the control section 24 in the second control method is described below using Fig. 10.

In this example of the operational procedure, first, an operator operates the touch-screen display 51 of the monitor 5 connected to the *in vivo* temperature control device 2 to preset, in the control section 24, any necessary value(s) selected from the first to Nth thresholds of the esophageal temperature, the first to Nth liquid-sending rates, the suction-starting cumulative amount of the sent liquid, and the suction rate. Here, the Nth liquid-sending rate is the rate at which the liquid is sent when the Nth threshold of the esophageal temperature is reached.

Subsequently, when the operator starts automatic operation, the control section 24 detects a signal(s) (such as the thermoelectromotive force) from the temperature sensor(s) 42 in the temperature probe 4 connected to the *in vivo* temperature control device 2, and then converts the signal(s) to temperature information (*in vivo* temperature(s)).

Subsequently, the control section 24 judges whether or not at least one *in vivo* temperature acquired has reached the preset first to Nth thresholds of the esophageal temperature, in other words, whether or not the condition for the start of sending of the liquid (cooling water) is satisfied. More specifically, in cases where the *in vivo* temperature has reached the (N-1)th threshold of the esophageal temperature, but has not reached the Nth threshold of the esophageal temperature, the control section 24 drives the pump 21 (forward rotation) to send the liquid at the (N-1)th liquid-sending rate. Here, in cases where the *in vivo* temperature has not reached the first threshold of the esophageal temperature, and the pump 21 is driven, the control section 24 stops the driving of the pump 21. In cases where the *in vivo* temperature has reached the Nth threshold of the esophageal temperature, the control section 24 drives the pump 21 to send the liquid at the Nth liquid-sending rate.

During the sending of the liquid, the control section 24 calculates the total amount of the liquid sent based on the liquid-sending rate and the liquid-sending time, and judges whether or not the total amount of the liquid sent has reached the suction-starting cumulative amount of the sent liquid. When the total amount of the liquid sent has reached the suction-starting cumulative amount of the sent liquid, the control section 24 changes the driving of the pump 21 from forward rotation to reverse rotation, to start suction of the liquid from the inside of the living body. After the start of the suction, the control section 24 calculates the internal tube pressure based on a signal detected by the pressure sensor 22, and continues the suction until the internal tube pressure becomes not more than the preset threshold of the suction stop pressure, that is, until the whole amount of the liquid is sucked or until the liquid becomes absent in the esophagus.

After the completion of the suction of the liquid, the control section 24 judges again whether or not the condition for the start of sending of the liquid (cooling water) is satisfied, and controls the *in vivo* temperature according to the above-described flow chart until the operator stops the automatic operation.

### EXAMPLES

A specific example of an *in vivo* temperature control system 1 of the present invention is described below with reference to drawings.

The *in vivo* temperature control system 1 of the present invention illustrated in Fig. 1 and Fig. 2 was prepared.

The pump 21, which is a roller-type tube pump used for both sending and sucking of the liquid, was provided such that the liquid-sending rate and the suction rate can be arbitrarily set within the range of 1 mL/min to 300 mL/min.

As the pressure sensor 22, the contact-type displacement gauge 221 was used. The pressure sensor 22 was provided such that the internal force of the liquid-sending-sucking dual-purpose tube 6 can be measured within the range of -80 kPaG to 150 kPaG.

As the liquid storage section 23, a 500-mL physiological saline bag was used. By cooling the physiological saline bag using the Peltier device 231, the temperature of the cooling water (physiological saline) was maintained within the range of 0°C to 10°C.

In the control section 24, a circuit capable of carrying out the first control method described above was incorporated. More specifically, according to a control method incorporated in the control section 24, when at least one of the temperatures obtained from the temperature sensors 42 has reached a preset threshold, the pump 21 is controlled such that the liquid is released from the liquid storage section 23 to the outside through the catheter 3 at a set liquid volume and flow rate. Further, a control method in which the pump 21 is controlled such that suction of the liquid is started when a set suction start time or suction start temperature or less is reached, and a control method in which the suction of the liquid is stopped when a set suction stop time is reached or when the pressure obtained from the pressure sensor 22 has reached a preset threshold of the suction stop pressure, were incorporated in the control section 24.

The tube section 31 of the catheter 3 has an outer diameter of 4.7 mm and an inner diameter of 3.3 mm. The valved connector 32 having the port 321 for connection to the liquid-sending-sucking dual-purpose tube 6 and having the valve 322 for fixation of the temperature probe 4 was placed in the handle part.

The shaft section 41 of the temperature probe 4 has an outer diameter of 2.0 mm. At the position 20 mm distant from the distal end side of the shaft section 41, six temperature sensors 42 were placed.

For the liquid-sending-sucking dual-purpose tube 6, a bottle needle for connection to the physiological saline bag; a three-way check valve for the channel-switch section; a tube section 31 for connection to the pump 21; a bulge section 61 and a contact-type displacement gauge 221 as the pressure sensor 22 for detection of the internal pressure of the liquid-sending-sucking dual-purpose tube 6; and a connection portion for connection to the catheter 3; were placed.

### (Experiment for Confirming Cooling Effect by Sending and Sucking of Cooling Water)

The temperature sensors 42 of the temperature probe 4 were inserted and fixed in the catheter 3 such that they protrude from the distal end portion, followed by placement in a simulated esophagus. Thereafter, the liquid-sending-sucking dual-purpose tube 6 was connected to the *in vivo* temperature control device 2 and the catheter 3, and set such that 15 mL of the cooling water is sent at a flow rate of 300 mL/min when the temperature of at least one of the six temperature sensors 42 has reached 38°C. In addition, the values of the suction start time, the suction start temperature, the amount of suction, the suction rate, and the suction stop pressure were set, and the operation was preliminarily set such that the cooling water is sucked at a flow rate of 60 mL/min after 30 seconds of sending of the liquid or when at least one of the six temperature sensors 42 has reached 36°C, and such that the suction of the cooling water is stopped when 20 seconds has passed after the start of the suction or when the pressure sensor 22 has reached -50 kPaG.

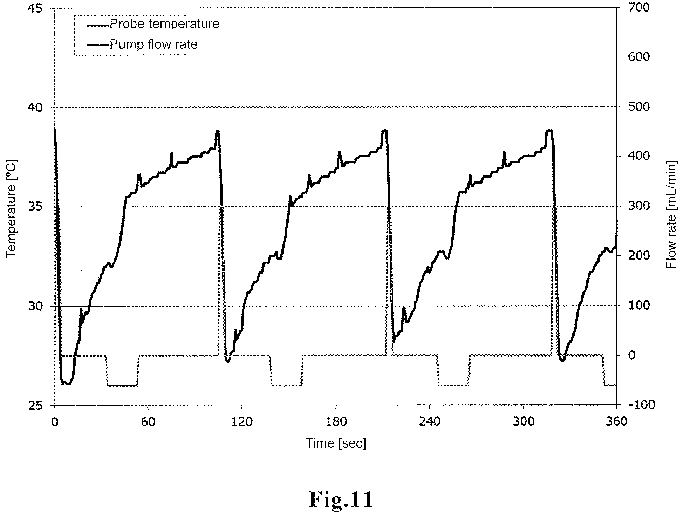

Fig. 11 illustrates the temperature of the simulated esophagus detected by the temperature probe 4, and the liquid-sending flow rate and the suction flow rate of the pump 21, as observed in a case where automatic operation of the *in vivo* temperature control system 1 was started after the internal temperature of the simulated esophagus was controlled to 40°C. When the temperature probe 4 is warmed to 38°C by the body temperature, the control section 24 judges that the signal detected from the temperature probe has exceeded the threshold, to start the liquid-sending operation by the pump 21 according to the operation programmed in advance. As a result, the cooling water is sent into the esophagus. It can be seen that the internal esophageal temperature was temporarily cooled to about 27 to 33°C as a result. Thereafter, the cooling water is sucked according to the operation programmed in advance, and the temperature probe 4 is warmed by the body temperature. Thus, the measured temperature of the temperature probe 4 gradually increases, and the cooling water is sent again when the temperature reaches 38°C. According to Fig. 11, it could be confirmed that the internal esophageal temperature can be constantly lowered to not more than 38°C by repeating of the sending and sucking operations.

### INDUSTRIAL APPLICABILITY

The present invention provides a system capable of automatically controlling the internal temperature of a body cavity upon changes in the internal temperature of the body cavity during an operation in the field of medicine

### DESCRIPTION OF SYMBOLS

*1: In vivo* temperature control system; 2: *in vivo* temperature control device; 3: catheter; 4: temperature probe; 5: monitor; 6: liquid-sending-sucking dual-purpose tube; 7: waste liquid section; 21: pump; 22: pressure sensor; 23: liquid storage section; 24: control section; 31: tube section; 32: valved connector; 41: shaft section; 42: temperature sensor; 43: handle section; 44: connection cable; 51: touch-screen display; 61: bulge section; 62: channel switching section; 63: liquid supply port; 64: connection port; 211: liquid-sending pump; 212: suction pump; 221: contact-type displacement gauge; 231: Peltier device; 321: port; 322: valve; 323: first port; 324: second port; 431: connector; 600: liquid-sending tube; 601: suction tube; 621: three-way check valve; 631: needle.

## Claims

1. An *in vivo* temperature control system comprising:
a catheter insertable into a living body;
a temperature probe containing a temperature sensor, the probe being insertable into the catheter;
a liquid storage section for storing a temperature-controlled liquid;
a pump for supplying the liquid from the liquid storage section to the catheter; and
a control section for controlling driving of the pump based on a signal detected from the temperature probe;
wherein
the control section controls the pump when the signal reaches a preset threshold, and
the pump is driven such that the liquid in the liquid storage section is released to the outside through the catheter.

2. The *in vivo* temperature control system according to claim 1, wherein
the control section controls the pump when a preset time or temperature is reached, and
the pump is driven such that a liquid in the outside is sucked through the catheter.

3. The *in vivo* temperature control system according to claim 1 or 2, comprising
a pressure sensor for detecting the internal pressure of the catheter, wherein
the control section controls driving of the pump based on a signal detected by the pressure sensor.

4. The *in vivo* temperature control system according to any one of claims 1 to 3, wherein
the control section drives the pump such that the rotation speed during the suction of the liquid is lower than the rotation speed during the sending of the liquid.

5. The *in vivo* temperature control system according to any one of claims 1 to 4, comprising
a monitor for displaying the signal detected from the temperature probe, as a digital number, bar graph, or trend graph,
the system having means for allowing an operator to know that the signal detected from the temperature probe exceeded the preset threshold, by way of changing display color of the digital number, bar graph, or trend graph on the monitor.

6. The *in vivo* temperature control system according to any one of claims 1 to 5, wherein
the pump is a liquid-sending pump and a suction pump, and
the control section drives the liquid-sending pump when the liquid is to be sent, and drives the suction pump when the liquid is to be sucked.

7. A method of controlling an *in vivo* temperature control system, the *in vivo* temperature control system comprising:
a catheter insertable into a living body;
a temperature probe containing a temperature sensor, the probe being insertable into the catheter;
a liquid storage section for storing a temperature-controlled liquid;
a pump for supplying the liquid from the liquid storage section to the catheter; and
a control section for controlling driving of the pump based on a signal detected from the temperature probe;
the method comprising the steps of:
making the control section compare the signal detected from the temperature probe with a preset threshold; and
making, when the signal is judged to have reached the threshold, the control section drive the pump such that the liquid in the liquid storage section is released to the outside through the catheter.
